Europäisches Patentamt

European Patent Office

Office européen des brevets

(11) Publication number: **0 323 144**
A2

# EUROPEAN PATENT APPLICATION

(21) Application number: 88312189.9

(22) Date of filing: 22.12.88

(51) Int. Cl.⁴ **B29C 47/02 , B29C 47/28 , B29C 47/24**

(30) Priority: 28.12.87 CS 9987/87
28.12.87 CS 9988/87
28.12.87 CS 9989/87

(43) Date of publication of application:
05.07.89 Bulletin 89/27

(84) Designated Contracting States:
DE FR GB NL

(71) Applicant: VYZKUMNY USTAV
POTRAVINARSKEHO PRUMYSLU
No. 21 Na belidle
Praha 5-Smichov(CS)

(72) Inventor: Cermak, Vlastimil
No. 2091 Tuklatska
Praha 10(CS)
Inventor: Krajicek, Milan
No. 30 Boleslavova
Praha 4(CS)
Inventor: Janovec, Ivan
No. 590 Podebradska
Praha 9(CS)
Inventor: Adam, Miloslav
No. 6 F. Sramka
Praha 5(CS)

(74) Representative: Hackett, Sean James et al
Marks & Clerk 57-60 Lincoln's Inn Fields
London WC2A 3LS(GB)

(54) **Method of manufacturing at least single-layer tubular blood vessel endoprosthesis, especially of a small internal diameter, and extruding nozzle for carrying out this method.**

(57) The method comprises a first phase wherein a thermoplastic biological material is extruded through an axial passage in a nozzle onto a core which is capable to reduce its profile if exposed to an axial tension whereby a homogeneous uniform layer of said material is created on said core, further a second phase wherein the layer of biological material is hardened and dried on said core, or, optionally, to the hardenen and dried layer at least another layer is applied and fixed thereon. and, finally, a third phase wherein the layer of biological material, or a laminate, respectively, contracts and becomes loose on the core by axially tensioning the latter so that the tubular layer can be easily removed therefrom.

FIG 1

# METHOD OF MANUFACTURING AT LEAST SINGLE-LAYER TUBULAR BLOOD VESSEL ENDOPROSTHESIS, ESPECIALLY OF A SMALL INTERNAL DIAMETER, AND EXTRUDING NOZZLE FOR CARRYING OUT THIS METHOD

The invention relates, on the one hand, to a method of manufacturing at least single-layer tubular blood vessel endoprosthesis, especially of a small internal diameter, and, on the other hand, to an extruding nozzle for carrying out the method.

As known, in the blood vessel surgical practice, there are successfully used tubular, single- or multi-layer blood vessel endoprostheses based on a biological absorbable material such as collagen, submucose, serose or the like. Multi-layer blood vessel prostheses usually comprise a non-absorbable, especially textile fabric layer made of synthetic fibers of e.g. polyester or polytetrafluoroethylene types. The layer of a textile fabric is applied onto the fundamental layer of biological material either in the form of a knitted hose to be drawn over said fundamental layer, or a strip of woven or knitted fabric is wrapped around the fundamental tubular layer.

Another method of manufacturing tubular blood vessel endoprostheses is based upon a superatmospheric pressure of a liquid medium of biological mass applied to the inner side of a texttile hose. Apart from this, a process is known, wherein a biological mass is applied onto a textile hose drawn over a stationary supporting core. After the biological mass has been hardened on the core, it is released thereon by rolling it on a hard support.

Many drawbacks of prior art as hereinabove referred to consist in a relative laboriousness, and particularly in irregular wall thickness and non-uniform tube surface, since the removal of the tube from the core frequently causes its deformation or damage. This particularly holds true with tubes whose length corresponds to about a centuple of their diameter.

It is an object of the present invention to eliminate the disadvantages of prior art as hereinabove set forth and to provide an improved method of manufacturing of at least single-layer blood vessel endoprostheses on the base of an absorbable biological material and having optimal functional parameters.

This object is substantially complied with by the method comprising a first phase wherein a thermoplastic biological material is extruded through an axial passage in a nozzle onto a core which is capable to reduce its profile if exposed to an axial tension, whereby a homogenous uniform layer of said material is created on said core, further a second phase wherein the layer of biological material is hardened and dried on said core, or, optionally, to the hardened and dried layer at least one another layer is applied and fixed thereon, and, finally, a third phase wherein the layer of biological material, or a laminate, respectively, becomes loose on the core by axially tensioning the latter so that the tubular layer can be easily removed therefrom.

Thus the core constitutes a support element for building a layer of biological material, or, optionally, for depositing other layers.

If necessary, a non-absorbable layer is applied to the layer of biological material hardened and dried on the core, said non-absorbable layer being then fixed thereon by means of another layer of biological material.

On the core, some additional technological steps can be effected such as, for instance, the surface of the layer of biological material, or of the laminate, respectively, is plastified by chemical means.

When forced under pressure through the extruding nozzle, the thermoplastic biological material is preferably shaped into the form of streams which are extruded through the cavity of the actual rotating extruding nozzle into its output portion and deposited, after leaving it, onto the core which is axially moved and rotated in the direction corresponding to the rotation of the actual rotating extruding nozzle.

If manufacturing tubular blood vessel endoprostheses, of greater lengths of, say, at least 400 mm, it is preferable to effect the first and the second phase on a core reinforced by means of a rod which is removed prior to the third phase. The support core which is made of a material capable of reducing its profile if axially tensioned, such as of a material based on an organic silicon compound, has a smooth, non-adhesive surface created e.g. by a hydrophobic coating.

For performing the method of the invention there is provided an extruding nozzle which, according to the invention, consists in that it comprises a head with a radial cavity communicating with a pressurized supply pipe for supplying the theremoplastic biological material, a guiding tube for a core, the tube being axially secured in said head, a hub which is fixedly attached to the head and in which an actual extruding nozzle is mounted for rotation, the nozzle being provided at its outer end with a pulley, further a distributor immovably disposed between the head and the hub and provided, on the one hand, with an axial opening in which the guiding tube is secured, and, on the other hand, with a system of holes which extend in parallel to said guiding tube, merge into said radial cavity of said head and open into the cavity of the actual rotating extruding nozzle, said guiding tube, which passes through said cavity of the actual rotating extruding nozzle, terminating in the nozzle output.

The core can be provided with an axial bore for a removable rod.

The method of the present invention allows the manufacture of tubular blood vessel endoprostheses having standard diameter, thickness and quality. It, however, enables also the tubes of extreme lengths of e,g. 1000 mm to be manufactured.

In order that the invention be better understood and carried into practice, a preferred embodiment thereof will hereinafter be described with reference to the accompanying schematic drawings in which

Fig. 1 shows an extruding nozzle, partially in an axial section;

Fig. 2 a support core reinforced by a rod, with a single layer of biological material on, in an axial section; and

Fig. 3 a support core with a laminated tube thereon, in an axial section.

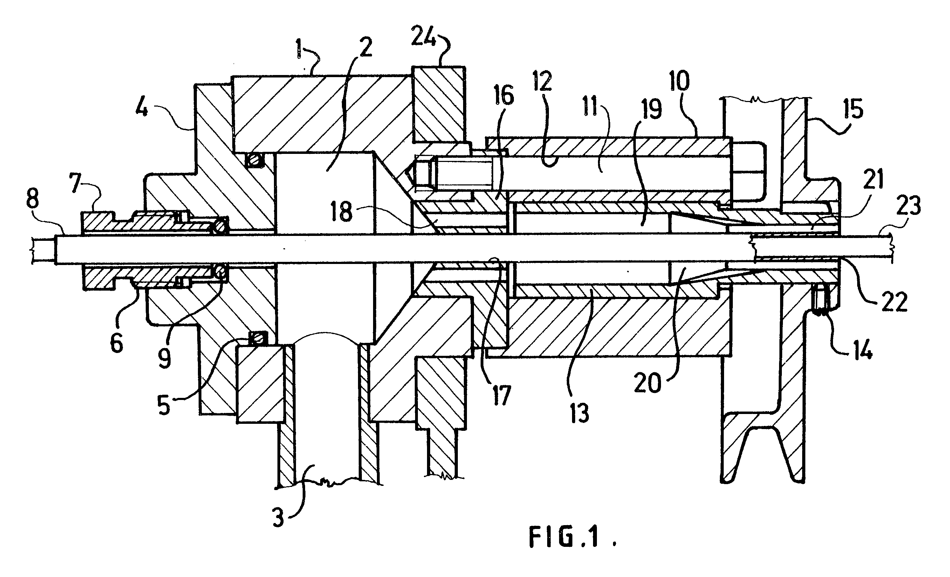

As can be seen in the drawings, and particularly Figure 1 thereof, the extruding nozzle comprises a head 1 with a radial cavity 2 communicating with a pressurized supply tube 3 for supplying a thermoplastic biological material from a receptacle (not shown). A lid 4 together with a sealing ring 5 is pressed on the head 1. In an axial threaded hold 6 of the lid 4 there is screwed a hollow screw 7 through which a guiding tube 8 extends, a packing 9 being provided between the screw 7 and a shoulder of said axial threaded hole 6.

By means of bolts 11 extending through longitudinal holes 12 in a hub 10, the hub 10 is secured to the head 1 and con stitutes a bearing for an actual rotating extruding nozzle 13. To the outer end of said nozzle 13 a pulley 15 is attached by a screw 14.

Between the radial cavity 2 and the actual rotating extruding nozzle 13 there is arranged a stationary distributor 16 for distributing the biological material. In an axial opening 17 provided in the distributor 16 the guiding tube 8 is pressed. The distributor 16 is provided with a system of e.g. seven holes 18 which extend in parallel to said guiding tube 2 and open into the cavity of the actual rotating extruding nozzle 13. This cavity consists of a cylindrical input portion 19 which merges over a bevel 20 to a cylindrical output portion 21. Through the guiding tube 8 which terminates in the output 22 of the actual rotating extruding nozzle 13 and protrudes somewhat from the hollow screw 7, the support core 23 extends. By means of a holder 24 the head 1 is secured to the machine frame (not shown).

The pulley is driven from an electric motor (not shown). The core 23 is given an axial and a rotational motion by not shown driving means associated with the extruding nozzle. The direction of its rotation corresponds to that of the actual rotating extruding nozzle 13, depending on its peripheral speed. Such means are not either described or shown in detail, since they are well-known in the art.

If manufacturing extremely long blood vessel endoprostheses it is preferable to use the support core 23 with an axial bore 25 for a removable rod 26.

The manufacture of the tubular blood vessel endoprosthesis proceeds as follows:

At first it is necessary to choose the dimension of the actual rotating extruding nozzle 13 at its cylindrical output portion 21 (e.g. 7 mm dia.), and to insert the support core 23 (of e.g. 4 mm dia. and 400 mm length) into the guiding tube 8.

Then the actual extruding nozzle 13 is set in rotation, the supply of thermoplastic biological material is started, and the core 23 is given the axial and rotary movement. After the material has filled the radial cavity 2 of the head 1, it is shaped, by forcing through holes 18 of the distributor 16, into strands which are then extruded through the cavity of the actual rotating nozzle 13 into its output portion 21 and deposited at its output 22 onto the rotating support core 23 so that a homogeneous and uniform layer of biological material is continuously formed thereon.

The above described first phase is followed by the second phase, wherein the layer is hardened and dried on the core.

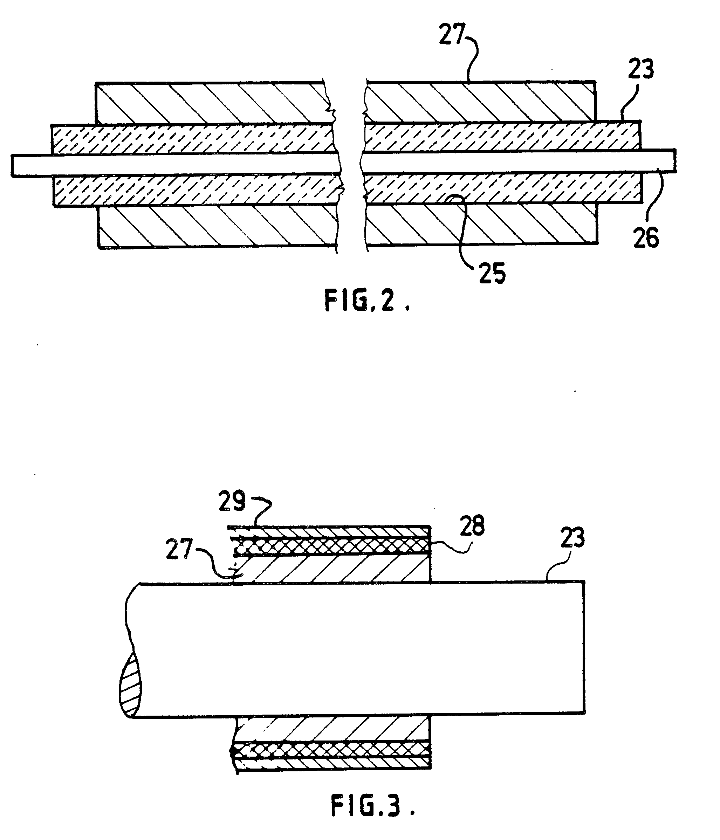

Fig. 2 shows an axial section of the support core 23 carrying a hardened layer 27 of biological material. Out of both ends of the core 23 protrudes the rod 26 inserted therein.

In the third phase, after the rod 26 has been pulled out of the core 23, the layer contracts and becomes loose on the core by pulling it at its both ends so that it can be easily removed from the core 23.

In this way a tube of 350 mm length can be obtained whereupon, after cutting off end portions, the final product of 300 mm length and 4 mm inner diameter is obtained.

If manufacturing combined or laminated blood vessel endoprostheses, a non-absorbable layer is applied onto the hardened and dried layer of biological material. Such a non-absorbable layer consisting, for example, of a tubular knitted fabric made of synthetic fibers, is drawn over the layer of biological material and fixed thereon by applying another layer of biological material which is subsequently hardened and dried.

Fig.3 shows an axial section of the support core 23 with a hardened layer 27 of biological material which is covered with a tubular knitted fabric 28 fixed by means of another layer 29 of biological material.

In the third phase, the combined or laminated layer on the core is released thereon by contracting and by pulling the core at both ends whereupon it is removed from it.

## EXAMPLE

The layer 29 of biological material, viz. collagen mass (pH 2.3; 6.5% dry matter content), was created by supplying the material through the extruding nozzle under pressure of 10 kp . cm⁻² and applying it onto the support core 23. The dried collagen layer was then hardened by dipping the core 23 for 15 minutes in one per cent aqueous solution of a suitable hardening resin. If necessary, the layer surface was plastified by dipping the core 23 in 35 per cent aqueous glycerol solution for 20 minutes.

The material was dried by suspending the core 23 in a drier at a temperature of from 15 to 25 °C until completely dry, which means for 24 hours. The temperature must not have exceeded 35 °C, since an elevated temperature would have endangered the desirable elasticity of the final tubular product.

Once dried, the tube on the core 23 contracted after the core had been axially tensioned, became loose and was removed from it. In the final phase the tube was dipped for 24 hrs. in heparin, put in a double-layered polyethylene wrapping and sealed. The sterilisation of the final product was effected by irradiating it by gamma radiation (2.7 Mrad).

## Claims

1. A method of manufacturing at least single-layer tubular blood vessel endoprosthesis, especially of a small internal diameter, on the basis of an absorbable biological material, the method being characterized in that it comprises a first phase wherein a thermoplastic biological material is extruded through an axial passage in a nozzle onto a core which is capable to reduce its profile if exposed to an axial tension/ whereby a homogeneous uniform layer of said material is created on said core, further a second phase wherein the layer of biological material is hardened and dried on said core, or, optionally, to the hardened and dried layer at least one other layer is applied and fixed thereon, and, finally, a third phase wherein the layer of biological material, or a laminate, respectively, becomes loose on the core by axially tensioning the latter so that the tubular layer can be easily removed therefrom.

2. A method according to claim 1, characterized in that to the layer of biological material hardened and dried on the core a non-absorbable layer is applied and then fixed thereon by means of another layer of biological material.

3. A method according to claim 1, characterized in that the surface of the layer of biological material, or of the laminate, respectively, on the core is plastified by chemical means.

4. A method according to claim 1, characterized in that the thermoplastic biological material, when forced under pressure through the extruding nozzle, is shaped into the form of streams which are extruded through the cavity of the actual rotating extruding nozzle into its output portion and deposited, after leaving it, onto the core which is axially moved and rotated in the direction corresponding to the rotation of the actual rotating extruding nozzle.

5. A method according to claim 1, characterized in that the first and the second phase are effected on a core reinforced by a rod.

6. An extruding nozzle for carrying out the method according to claims 1 through 4, characterized in that it comprises a head (1-) with a radial cavity (2) communicating with a pressurized supply pipe (3) for supplying the thermoplastic biological material, a guiding tube (8) for a core (23), the tube (8) being axially secured in said head (1), a hub (10) which is fixedly attached to the head (1) and in which an actual extruding nozzle (13) is mounted for rotation, the nozzle (13) being provided at its outer end with a pulley (15), further a distributor (16) immovably disposed between the head (1) and the hub (10) and provided, on the one hand, with an axial opening (17) in which the guiding tube (8) is secured, and, on the other hand, with a system of holes (18) which extend in parallel to said guiding tube (8), merge into said radial cavity (2) of said head (1) and open into the cavity of the actual rotating extruding nozzle (13), said guiding tube (8), which passes through said cavity of the actual rotating extruding nozzle (13), terminating in the nozzle output (22).

7. An extruding nozzle according to claim 6, characterized in that the core (23) is provided with an axial bore (25) for a removable rod (26).

FIG.1.

FIG. 2 .

FIG. 3 .